# EUROPÄISCHE PATENTSCHRIFT

(11) **EP 0 332 971 B1**
(45) Veröffentlichungstag und Bekanntmachung des Hinweises auf die Patenterteilung: **15.09.1993**
(21) Anmeldenummer: 89103908.3
(22) Anmeldetag: 06.03.1989
(51) Int. Cl.: C07C 69/30, C07C 67/03

(54) **Verfahren zur kontinuierlichen Umesterung von Fettsäureniedrigalkyl-estern**
Process for the transesterification of short chain alkyl esters of fatty acids
Procédé de transestérification continue d'esters d'alkyle à courte chaîne et d'acides gras

(30) Priorität: 14.03.1988 DE 3808427
(43) Veröffentlichungstag der Anmeldung: 20.09.1989
(73) Patentinhaber: Henkel Kommanditgesellschaft auf Aktien, 40191 Düsseldorf (DE)
(72) Erfinder: Jeromin, Lutz, Dr., D-4010 Hilden (DE); Peukert, Eberhard, D-4010 Hilden (DE); Gutsche, Bernhard, Dr., D-4010 Hilden (DE); Wollmann, Gerhard, Dr., D-4010 Hilden (DE); Schleper, Bernhard, Dr., D-4000 Düsseldorf 1 (DE)

(56) Entgegenhaltungen:
- EP-A- 0 291 870

## Beschreibung

Die Erfindung betrifft ein Verfahren zur kontinuierlichen Umesterung von C₆-C₂₂-Fettsäure-niedrigalkyl-estern mit mehrwertigen C₂-C₅-Alkoholen unter homogener alkalischer Katalyse.

Umsetzungen von Methylestern von Fettsäuren mit mehrwertigen Alkoholen, insbesondere Glycerin, werden bisher hauptsächlich in diskontinuierlichen bzw. semikontiniuerlichen Anlagen durchgeführt. Die dabei erhaltenen Endprodukte, insbesondere Triglyceride, können als solche einer Vielfalt von technischen Anwendungen zugeführt werden. Erhaltene Glyceride und Partialglyceride können mit Wasser unter Druck in Fettsäuren überführt werden. Der gegenüber der direkten Verseifung von Fettsäureestern damit beschrittene Umweg über die Glyceride ist aus Gründen der Abwasseraufbereitung vorteilhaft, weil hier keine Salzbelastung im Abwasser auftritt.

Umesterungsreaktionen von Fettsäuremethylestern mit Monoalkoholen in kontinuierlich arbeitenden Reaktionskolonnen sind in der EP-B 0082 301 erwähnt, ohne daß dabei jedoch auf Verfahrenseinzelheiten Bezug genommen wird. Eine Übersicht über die Umesterung von Fettsäuremethylestern mit Glycerin ist in JAOCS 59, Vol. 10, 795A - 802A (1982) veröffentlicht.

Das Verfahren der Erfindung ist dadurch gekennzeichnet, daß man
a) eine mit einer Mehrzahl von Böden ausgestattete Reaktionskolonne mit nachgeschaltetem Rektifikationsteil verwendet, die in ihrem unteren Kolonnenteil im Siedegleichgewicht und in ihrem oberen Teil überwiegend in einem Absorptions-/Desorptions-Gleichgewicht betrieben wird,
b) vorerhitzte, mehrwertige C₂-C₅-Alkohole und alkalische, in einem Lösemittel gelöste Katalysatoren, ausgenommen Natriumcarbonat, auf den oberen Teil der Reaktionskolonne gibt,
c) siedenden bzw. überhitzten Fettsäure-niedrigalkyl-ester auf den mittleren Teil der Reaktionskolonne gibt,
d) am Rektifikationsteil in der Kolonne gebildete bzw. mit dem Katalysator eingebrachte niedrige Alkanole rektifiziert und
e) im Kolonnensumpf gesammeltes, im wesentlichen aus Fettsäure-niedrigalkyl-estern, mehrwertigem Alkohol und Fettsäureestern mit den mehrwertigen Alkoholen bestehendes Produkt abzieht und teilweise nach Erhitzen auf eine Temperatur oberhalb der Sumpftemperatur in den unteren Teil der Reaktionskolonne zurückführt sowie den übrigen Teil des Produktes in üblicher Weise aufarbeitet.

Mit dem Verfahren der Erfindung können sämtliche in der Fettchemie üblichen C₆-C₂₂-Fettsäure-niedrigalkyl-ester mit 1 bis 5 Kohlenstoffatomen in der Alkoholkomponente, z.B. Ethyl-, Propyl-, Butyl- oder Pentylester, bevorzugt jedoch die Methylester von Fettsäuren natürlichen, insbesondere pflanzlichen und tierischen oder seetierischen sowie synthetischen Ursprungs umgeestert werden, einschließlich der Methylester von technischen Fettsäuregemischen, wie sie beispielsweise aus Talg, Kokosöl, Sojaöl, Palmöl und sonstigen pflanzlichen und tierischen Fetten zugänglich sind. Speziell zu nennen sind die bei der Fettspaltung anfallenden Methylester der sogenannten Vorlauffettsäuren mit Kettenlängen von C₆-C₁₂.

In dem Verfahren der Erfindung können grundsätzlich sämtliche Bodenkolonnen mit einer geeigneten Anzahl von Böden, z.B. Siebböden, eingesetzt werden. Besonders geeignet sind Glockenböden mit hohen Flüssigkeitsständen, insbesondere Doppelglockenböden, wie sie z.B. aus der bereits genannten EP-B 0082 301 sowie aus der DE-C 25 03 195 und der EP-B 0033 929 bekannt sind.

Die Aufarbeitung des am Sumpf der Reaktionskolonne abgezogenen Produktes erfolgt mittels üblicher Methoden, insbesondere durch Destillation.

Als im Verfahren der Erfindung einzusetzende C₂-C₅-Alkohole können Ethylenglykol, Propylenglykol, Glycerin, Trimethylolpropan und Pentaerythrit genannt werden; bevorzugt ist die Umsetzung mit Glycerin.

Als Katalysatoren werden in dem Verfahren der Erfindung die für Umesterungsreaktionen üblichen eingesetzt, insbesondere Alkalialkoholate und -hydroxide aus der von Lithiumhydroxid, Natriumhydroxid, Natriummethylat und Kaliumhydroxid gebildeten Gruppe; die Verwendung von Natriumhydroxid und Natriummethylat ist bevorzugt. Als Lösemittel für die erfindungsgemäß eingesetzten Katalysatoren eignen sich insbesondere diejenigen mehrwertigen Alkanole, die zur Umesterung verwendet werden, oder diejenigen entsprechend der Alkoholkomponente der umzuesternden Fettsäureester. Katalysatoren wie NaOH und KOH können gegebenenfalls auch in geringen Mengen Wasser gelöst eingesetzt werden.

Gemäß einer vorteilhaften Ausführungsform der Erfindung betreibt man die Reaktionskolonne bei einem Kopfdruck von 200 - 700, insbesondere von 200 - 300 hPa.

Gemäß einer weiteren vorteilhaften Ausführungsform der Erfindung gibt man die Fettsäure-niedrigalkyl-ester mit Temperaturen von 140 - 240, insbesondere 180 bis 200 °C, und Glycerin bei einer Temperatur von 140 - 200, insbesondere 160 - 180 °C, auf und speist das in den unteren Teil der Raktionskolonne zurückgeführte, im wesentlichen aus Fettsäure-niedrigalkyl-ester, Glycerin und Fettsäureglyceriden bestehende Produkt mit Temperaturen ein, daß sich im Kolonnensumpf eine Temperatur zwischen 180 und 240, insbesondere zwischen 200 und 220 °C einstellt. Im übrigen wird die Kolonne in ihrem mittleren Bereich bei Temperaturen von 140 - 200 °C und in ihrem Kopfbereich von 30 - 150 °C betrieben.

Der in Abweichung von üblichen Veresterungsverfahren vorgesehene Zwangsumlaufverdampfer im Sumpf der Kolonne gewährleistet eine ausreichende Dampfbelastung auch im unteren Reaktorteil. Da weiterhin statt der bei Veresterungsverfahren üblichen Zugabe von überhitzten Sumpfprodukten die leichter flüchtige Komponente (Fettsäure-niedrigalkyl-ester) dem Reaktor siedend zugeführt wird, arbeitet die Reaktionskolonne im unteren Kolonnenteil im Siedegleichgewicht; dagegen liegt im oberen Teil überwiegend ein Absorptions-/Desorptions-Gleichgewicht vor.

Infolge der Zugabe des Katalysators in flüssiger Phase an den Kopf der Kolonne sinkt die Reaktortemperatur auf den obersten Böden deutlich ab. Daher stellen sich die bereits genannten Kopftemperaturen zwischen 30 und 150 °C ein.

Der nachgeschaltete Rektifikationsteil der Reaktionskolonne, der zweckmäßigerweise in Form eines Rektifikationsaufsatzes ausgebildet ist, muß hinsichtlich der Zahl seiner Böden so bemessen sein, daß als Kopfprodukt der Rektifikation reines Methanol zurückgewonnen wird, so daß die sonst übliche Aufarbeitung des Destillats entfallen kann; hierzu ist die Einstellung eines entsprechenden Rücklaufverhältnisses für Methanol erforderlich, dessen Auswahl für den Fachmann jederzeit möglich ist.

Gemäß einer weiteren vorteilhaften Ausführungsform der Erfindung setzt man Fettsäure-niedrigalkyl-ester und Glycerin - in Abhängigkeit von den gewünschten Endprodukten - in molaren Verhältnissen von 1:1 bis 10:1 um. Vorzugsweise verwendet man bei der Herstellung von überwiegend Triglyceride enthaltenden Produkten Fettsäure-niedrigalkyl-ester und Glycerin in molaren Verhältnissen von 3:1 bis 10:1 um. Will man dagegen überwiegend Partialglyceride herstellen, beträgt das bevorzugte molare Verhältnis von Fettsäure-niedrigalkyl-ester zu Glycerin 1:1 bis 3:1, insbesondere 1,3 bis 2:1 und - besonders bevorzugt - 1,4:1 bis 1,8:1. Bei anderen, nicht trifunktionellen mehrwertigen Alkanolen ändern sich die molaren Verhältnisse entsprechend ihrer Funktionalität.

Gemäß einer weiteren vorteilhaften Ausführungsform der Erfindung arbeitet man bei der Umesterung mit Verweilzeiten, bezogen auf eingesetztes Glycerin, von 3 bis 40 Stunden.

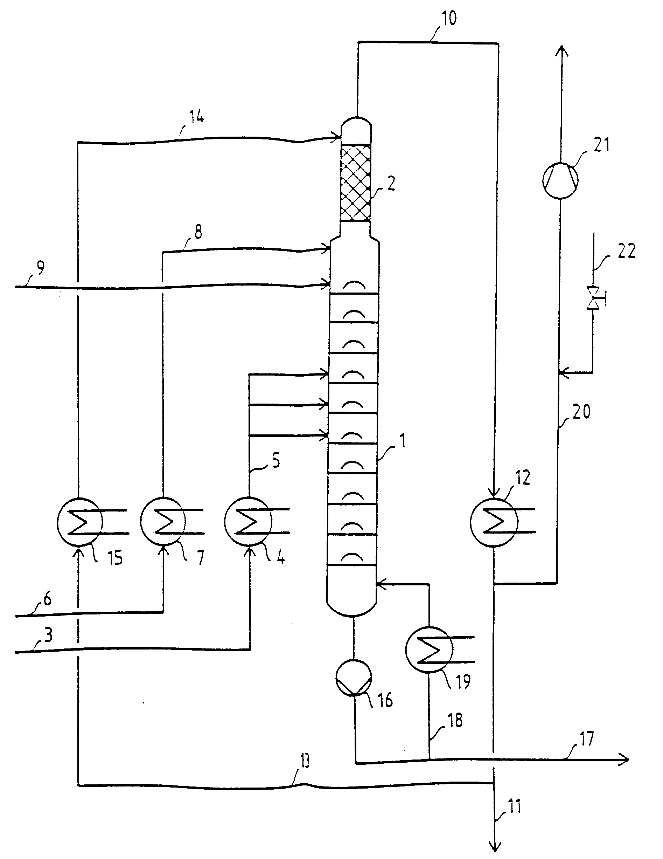

Die Erfindung wird im folgenden anhand einer Beschreibung einer bevorzugten Anlage näher erläutert, wobei auf die Zeichnung Bezug genommen wird, welche ein Verfahrensschema zur kontinuierlichen Umesterung z.B. von Fettsäuremethylestern zeigt.

Gemäß der Zeichnung umfaßt die dargestellte Anlage eine Reaktionskolonne 1 mit einem Durchmesser von 36 cm und 30 Glockenböden jeweils 10 Glocken. Der auf die Reaktionskolonne aufgesetze Rektifikationsteil 2 besteht aus 2 Drahtgewebepackungen der Firma Montz (je 1,1 m hoch). Der umzusetzende Fettsäuremethylester wird über eine Leitung 3 einem Wärmetauscher 4 zugeführt und durch Erhitzen in einen siedenden bzw. überhitzten Zustand überführt. Der Fettsäuremethylester wird anschließend über eine Leitung 5 an dem mittleren Teil der Reaktionskolonne aufgegeben. Das umzusetzende Glycerin wird über eine Leitung 6 einem zweiten Wärmeaustauscher 7 zugeführt, dort erhitzt und über eine Leitung 8, ebenso wie die Katalysatorlösung über eine Leitung 9, am obersten Boden der Reaktionskolonne aufgegeben. Am Kopf des Rektifikationsteils 2 abgenommenes Methanol wird teilweise über Leitungen 10 und 11 nach Kühlen in einem dritten Wärmeaustauscher 12 aus dem System entfernt und teilweise über Leitungen 13 und 14 mit zwischengeschaltetem Wärmeaustauscher 15 als Rücklauf an den Kopf des Rektifikationsteils zurückgegeben.

Das im Sumpf der Reaktionskolonne sich sammelnde Produkt wird mit Hilfe einer Pumpe 16 teilweise über eine Leitung 17 aus dem System entfernt und zum anderen Teil über eine Rücklaufleitung 18 und nach Erhitzen auf eine Temperatur oberhalb der Sumpftemperatur mittels eines fünften Wärmeaustauschers 19 in den unteren Teil der Reaktionskolonne zurückgeführt. Die Evakuierung des Systems erfolgt über eine Leitung 20 und eine Vakuumpumpe 21, die Druckregelung mittels einer Stickstoffleitung 22.

Die Erfindung wird im folgenden anhand bevorzugter Ausführungsbeispiele näher erläutert.

### Beispiel 1:

Umesterung eines Kokosvorlaufmethylesters (ca .1 % C₆; 59 % C₈; 39 % C₁₀; 1 % C₁₂), im folgenden mit VME bezeichnet.

Die Reaktionsparameter waren wie folgt:
Molares Verhätnis VME/Glycerin 1,5 :1
Einsatztemperatur Glycerin 170 °C
Einsatztemperatur VME 200 °C
Kopfdruck in der Reaktionskolonne 250 hPA
Sumpftemperatur 230°C;
Verhältnis von abgezogenem Sumpfprodukt zu rückgeführtem Sumpfprodukt 1:15;
Temperatur des rückführten Sumpfproduktes beim Eintritt in die Reaktionskolonne 235 bis 245°C;
mittlere Verweilzeit des Glycerins ca. 8 Stunden;
Zusammensetzung des Sumpfproduktes:
Glycerin 1,9 %, VME 6,5 %, Rest Fettsäureglyceride.

### Beispiel 2:

Das Beispiel 1 wurde mit einem molaren Verhältnis von VME/Glycerin von 1,8:1 wiederholt, die übrigen Temperatur- und Druckparameter blieben unverändert. Das Sumpfprodukt enthielt 2,5 % Glycerin und 9 % VME, Rest Fettsäureglyceride.

## Patentansprüche

1. Verfahren zur kontinuierlichen Umesterung von C₆-C₂₂-Fettsäure-niedrigalkyl-estern mit mehrwertigen C₂-C₅-Alkoholen unter homogener alkalischer Katalyse, dadurch gekennzeichent, daß man
a) eine mit einer Mehrzahl von Böden ausgestattete Reaktionskolonne mit nachgeschaltetem Rektifikationsteil verwendet, die in ihrem unteren Kolonnenteil im Siedegleichgewicht und in ihrem oberen Kolonnenteil überwiegend in einem Absorptions-/Desorptions-Gleichgewicht betrieben wird,
b) vorerhitzte, mehrwertige C₂-C₅-Alkohole und alkalische, in einem Lösemittel gelöste Katalysatoren, ausgenommen Natriumcarbonat, auf den oberen Teil der Reaktionskolonne gibt,
c) siedenden Fettsäure-niedrigalkyl-ester auf den mittleren Teil der Reaktionskolonne gibt.
d) am Rektifikationsteil in der Kolonne gebildete niedrige Alkanole rektifiziert,
e) im Kolonnensumpf gesammeltes Produkt, im wesentlichen bestehend aus Fettsäure-niedrigalkyl-ester, mehrwertigem Alkohol und Fettsäureester mit den mehrwertigen Alkoholen, abzieht und teilweise nach Erhitzen auf eine Temperatur oberhalb derjenigen der Sumpftemperatur in den unteren Teil der Reaktionskolonne zurückführt sowie den übrigen Teil des Sumpfproduktes in üblicher Weise aufarbeitet.

2. Verfahren nach Anspruch 1, dadurch gekennzeichnet, daß man die Fettsäure-niedrigalkyl-ester mit Glycerin umestert.

3. Verfahren nach Anspruch 1 oder 2, dadurch gekennzeichnet, daß man Fettsäuremethylester umestert.

4. Verfahren nach mindestens einem der Ansprüche 1 bis 3, dadurch gekennzeichnet, daß man Katalysatoren aus der von Lithiumhydroxid, Natriumhydroxid, Natriummethylat und Kaliumhydroxid gebildeten Gruppe verwendet.

5. Verfahren nach mindestens einem der Ansprüche 1 bis 4, dadurch gekennzeichnet, daß man die Reaktionskolonne bei einem Kopfdruck von 200 - 700, insbesondere von 200 - 300 hPa, betreibt.

6. Verfahren nach mindestens einem der Ansprüche 1 bis 5, dadurch gekennzeichnet, daß man die Fettsäure-niedrigalkyl-ester mit Temperaturen von 140 - 240, insbesondere 180 - 200 °C, und Glycerin bei einer Temperatur von 140 - 200, insbesondere 160 - 180 °C, auf die Reaktionskolonne aufgibt und das in den unteren Teil der Reaktionskolonne zurückgeführte, im wesentlichen aus Fettsäure-niedrigalkyl-ester, Glycerin und Fettsäureglyceriden bestehende Produkt mit Temperaturen einspeist, daß sich im Kolonnensumpf eine Temperatur zwischen 180 und 240, insbesondere zwischen 200 und 220 °C, einstellt.

7. Verfahren nach mindestens einem der Ansprüche 1 bis 6, dadurch gekennzeichnet, daß man die Umesterung bei Temperaturen von 140 - 200 °C im mittleren Bereich und von 30 - 150 °C im Kopfbereich der Reaktionskolonne durchführt.

8. Verfahren nach mindestens einem der Ansprüche 1 bis 7, dadurch gekennzeichnet, daß man Fettsäure-niedrigalkyl-ester und Glycerin in molaren Verhältnissen von 1:1 bis 10:1 umsetzt.

9. Verfahren nach mindestens einem der Ansprüche 1 bis 8, dadurch gekennzeichnet, daß man zur Herstellung von triglyceridreichen Produkten Fettsäure-niedrigalkyl-ester und Glycerin in molaren Verhältnissen von 3:1 bis 10:1 umsetzt.

10. Verfahren nach mindestens einem der Anprüche 1 bis 9, dadurch gekennzeichnet, daß man zur Herstellung von partialglyceridreichen Produkten Fettsäure-niedrigalkyl-ester und Glycerin in molaren Verhältnissen von 1:1 bis 3:1 umsetzt.

11. Verfahren nach Anspruch 10, dadurch gekennzeichnet, daß man zur Herstellung von partialglyceridreichen Produkten Fettsäure-niedrigalkyl-ester und Glycerin in molaren Verhältnissen von 1,3:1 bis 2:1 umsetzt.

12. Verfahren nach Anspruch 10 oder 11, dadurch gekennzeichnet, daß man zur Herstellung von partialglyceridreichen Produkten Fettsäure-niedrigalkyl-ester und Glycerin in molaren Verhältnissen von 1,4:1 bis 1,8:1 umsetzt.

13. Verfahren nach mindestens einem der Ansprüche 1 bis 12, dadurch gekennzeichnet, daß man die Umesterung bei Verweilzeiten, bezogen auf eingesetztes Glycerin, von 3 bis 40 Stunden durchführt.

## Claims

1. A process for the continuous transesterification of C₆-C₂₂ fatty acid lower alkyl esters with polyhydric C₂-C₅ alcohols in the presence of homogeneous alkaline catalysts, characterized in that
a) a multiple-plate reaction column followed by a rectifying section is used, being operated in the boiling equilibrium in its lower section and predominantly in an absorption/desorption equilibrium in its upper section,
b) preheated polyhydric C₂-C₅ alcohols and alkaline catalysts, except sodium carbonate, dissolved in a solvent are introduced into the upper part of the reaction column,
c) boiling fatty acid lower alkyl esters are introduced into the middle part of the reaction column,
d) lower alkanols formed in the column are rectified in the rectifying section and
e) product collected in the sump of the column and consisting essentially of fatty acid lower alkyl ester, polyhydric alcohol and fatty acid ester with the polyhydric alcohols is removed and, after heating to a temperature above the sump temperature, is partially returned to the lower part of the reaction column while the rest of the sump product is worked up in the usual way.

2. A process as claimed in claim 1, characterized in that the fatty acid lower alkyl ester is transesterified with glycerol.

3. A process as claimed in claim 1 or 2, characterized in that fatty acid methyl ester is transesterified.

4. A process as claimed in at least one of claims 1 to 3, characterized in that catalysts from the group consisting of lithium hydroxide, sodium hydroxide, sodium methylate and potassium hydroxide are used.

5. A process as claimed in at least one of claims 1 to 4, characterized in that the reaction column is operated at a head pressure of from 200 to 700 hPa and more especially from 200 to 300 hPa.

6. A process as claimed in at least one of claims 1 to 5, characterized in that the fatty acid lower alkyl esters are introduced into the reaction column at temperatures of 140 to 240°C and more especially at temperatures of 180 to 200°C and the glycerol at a temperature of 140 to 200°C and more especially at a temperature of 160 to 180°C while the product consisting essentially of fatty acid lower alkyl ester, glycerol and fatty acid glycerides, which is returned to the lower part of the reaction column, is introduced at such temperatures that a temperature of from 180 to 240°C and more especially from 200 to 220°C is established in the column sump.

7. A process as claimed in at least one of claims 1 to 6, characterized in that the transesterification is carried out at temperatures in the range from 140 to 200°C in the middle part of the reaction column and at temperatures in the range from 30 to 150°C at the head of the reaction column.

8. A process as claimed in at least one of claims 1 to 7, characterized in that fatty acid lower alkyl ester and glycerol are reacted in a molar ratio of 1 : 1 to 10 : 1.

9. A process as claimed in at least one of claims 1 to 8, characterized in that, to produce products predominantly containing triglycerides, fatty acid lower alkyl ester and glycerol are reacted in a molar ratio of 3 : 1 to 10 : 1.

10. A process as claimed in at least one of claims 1 to 9, characterized in that, to produce products predominantly containing partial glycerides, fatty acid lower alkyl ester and glycerol are reacted in a molar ratio of 1 : 1 to 3 : 1.

11. A process as claimed in claim 10, characterized in that, to produce products rich in partial glycerides, fatty acid lower alkyl ester and glycerol are reacted in a molar ratio of 1.3 : 1 to 2 : 1.

12. A process as claimed in claim 10 or 11, characterized in that, to produce products predominantly containing partial glycerides, fatty acid lower alkyl ester and glycerol are reacted in a molar ratio of 1.4 : 1 to 1.8. : 1.

13. A process as claimed in at least one of claims 1 to 12, characterized in that the residence times during the transesterification, based on the glycerol used, are from 3 to 40 hours.

## Revendications

1. Procédé de transestérification continue d'esters alkyliques inférieurs d'acides gras de C₆ à C₂₂ avec des polyalcools de C₂ à C₅, sous catalyse alcaline homogène, caractérisé en ce que
a) l'on utilise une colonne de réaction pourvue de plusieurs fonds ou plateaux, avec une partie de rectification intercalée à la suite, qui est opérée dans sa partie inférieure en équilibre d'ébullition et dans sa partie supérieure, en majeure partie, en équilibre d'absorption/désorption.
b) l'on verse des polyalcools préchauffés de C₂ à C₅ et des catalyseurs alcalins, à l'exception du carbonate de sodium, dissous dans un solvant, sur la partie supérieure de la colonne de réaction,
c) l'on verse l'ester alkylique inférieur d'acide gras en ébullition sur la partie moyenne de la colonne de réaction,
d) l'on rectifie dans la partie de rectification les alcanols inférieurs formés dans la colonne,
e) l'on soutire le produit, accumulé dans le fond de la colonne, qui est essentiellement constitué d'esters alkyliques inférieurs d'acides gras, de polyalcool et d'esters d'acide gras, avec les polyalcools et en ce qu'on le recycle dans la partie inférieure de la colonne de réaction, en partie après chauffage à une température supérieure à celle de la température du fond et en ce que l'on retraite la partie restante du produit de fond de la manière habituelle.

2. Procédé selon la revendication 1, caractérisé en ce que l'on transestérifie les esters alkyliques inférieurs d'acides gras avec de la glycérine.

3. Procédé selon la revendication 1 ou 2, caractérisé en ce que l'on transestérifie de l'ester méthylique d'acide gras.

4. Procédé selon au moins une des revendications 1 à 3, caractérisé en ce que l'on utilise des catalyseurs faisant partie du groupe formé par l'hydroxyde de lithium, l'hydroxyde de sodium, le méthylate de sodium et l'hydroxyde de potassium.

5. Procédé selon au moins une des revendications 1 à 4, caractérisé en ce que l'on opère la colonne de réaction à une pression de tête de 200 à 700, en particulier de 200 à 300 hPa.

6. Procédé selon au moins une des revendications 1 à 5, caractérisé en ce que l'on verse les esters alkyliques inférieurs d'acides gras à des températures de 140 à 240, en particulier de 180 à 200 °C, et la glycérine à une température de 140 à 200, en particulier de 160 à 180 °C, sur la colonne de réaction et en ce l'on introduit le produit recyclé dans la partie inférieure de la colonne de réaction, essentiellement constitué d'ester alkylique inférieur d'acide gras, de glycérine et de glycérides d'acide gras, à des températures telles que la température dans le fond de la colonne s'établit entre 180 et 240, en particulier entre 200 et 220 °C.

7. Procédé selon au moins une des revendications 1 à 6, caractérisé en ce que l'on exécute la transestérification à des températures comprises entre 140 et 200 °C, dans la zone moyenne et entre 30 et 150 °C, dans la zone de la tête de la colonne de réaction.

8. Procédé selon au moins une des revendications 1 à 7, caractérisé en ce que l'on fait réagir les esters alkyliques inférieurs d'acides gras et la glycérine dans des rapports molaires compris entre 1:1 et 10:1.

9. Procédé selon au moins une des revendications 1 à 8, caractérisé en ce que pour préparer des produits riches en triglycérides, on fait réagir les esters alkyliques inférieurs d'acides gras et la glycérine dans des rapports molaires compris entre 3:1 et 10:1.

10. Procédé selon au moins une des revendications 1 à 9, caractérisé en ce que pour préparer des produits riches en glycérides partiels, on fait réagir les esters alkyliques inférieurs d'acides gras et la glycérine dans des rapports molaires compris entre 1:1 et 3:1.

11. Procédé selon au moins une des revendications 1 à 10, caractérisé en ce que pour préparer des produits riches en glycérides partiels, on fait réagir les esters alkyliques inférieurs d'acides gras et la glycérine dans des rapports molaires compris entre 1,3:1 et 2:1.

12. Procédé selon la revendication 10 ou 11, caractérisé en ce que pour préparer des produits riches en glycérides partiels, on fait réagir les esters alkyliques inférieurs d'acides gras et la glycérine dans des rapports molaires compris entre 1,4:1 et 1,8:1.

13. Procédé selon au moins une des revendications 1 à 12, caractérisé en ce que l'on exécute la transestérification pendant des temps de séjour allant de 3 à 40 heures, en fonction de la glycérine mise en oeuvre.
